# EUROPEAN PATENT APPLICATION

(11) **EP 2 233 157 A1**
(43) Date of publication of application: **29.09.2010**
(21) Application number: 08862032.3
(22) Date of filing: 08.12.2008
(51) Int. Cl.: A61L 15/28, A61K 31/715

(54) **A BIOCOMPATIBLE DENATURED STARCH SPONGE MATERIAL**

(30) Priority: 11.12.2007 CN 200710199682
(71) Applicant: Ji, Xin, Shanghai 200335 (CN)
(72) Inventor: Ji, Xin, Shanghai 200335 (CN)
(74) Representative: Lang, Christian
(86) International application number: PCT/CN2008/073375
(87) International publication number: WO 2009/076873

(57) **Abstract**

The present invention relates to a biocompatible modified starch hemostatic sponge/foam and its new use as any of the hemostat, anti-adhesion material, promoting tissue healing material, surgical sealant and tissue adhesive glue. The said modified starch includes any or a composite of more than one of the pregelatinized starch, acid modified starch, oxidized starch, esterified starch, etherized starch, cross-linked starch, and grafted starch, composite modified starch.

The said hemostatic sponge/foam in this patent is made from modified starch, or a combination of modified starch with other biocompatible hemostat, blood coagulants, plasticizer etc. via vacuum freeze drying process. The advantages of the modified starch hemostatic foam/sponge include 1) multimodality - it can be applied directly to bleeding wound site; 2) having super biocompatibility which can minimize the risks of anaphylaxis, wound infection and wound healing retardation resulting from applying other animal/human components derived hemostats; 3) having super adhesive properties following water absorption which can create the adhesive matrix of clot to adhere and seal the bleeding vessels and wound tissue in particular when profuse bleeding.

## Description

### Field of Invention

The present invention relates to a biocompatible modified starch derived hemostatic sponge/foam which can be applied directly to the wound surface of humans and mammals. The present invention also relates to the said biocompatible modified starch hemostatic sponge/foam and its application as a hemostatic material, biocompatible anti-adhesion material, tissue healing promotion agent, absorbable surgical sealant and tissue adhesive glue.

### Description of Related Arts

Surgical operations and trauma may create bleeding wounds, which can produce a risk of excess blood loss. Therefore, hemostats to control bleeding should be applied in timely manner. It is common to apply biocompatible absorbable hemostatic agents to bleeding wound sites to achieve hemostasis in surgical procedures, trauma treatment and home self rescue. There is clinical benefit to provide patients a hemostatic agent which is safe, efficacious, easy to use and cost effective.

Prior absorbable hemostats consist of the following classes of materials:
1. Hemostatic sponge class: gelatin sponge, collagen sponge, chitosan sponges, carboxymethyl cellulose sponge, thrombin and fibrin sponges.
2. Hemostatic gauze / hemostatic film class: oxidized cellulose gauze, oxidized regenerated cellulose gauze.
3. Hemostatic glues class: fibrin glue, synthetic glue.
4. Polysaccharide hemostatic powder class: microporous polysaccharide powder, chitosan powder.

A detailed analysis of the biocompatible hemostat in common use is stated below:

### 1. Absorbable gelatin sponges and collagen sponges

The gelatin sponge is extracted from animal tissue, and the main component of the gelatin sponge is animal collagen. The gelatin sponge has a hydrophilic and multi-porous structure to concentrate blood components by absorbing water in the blood to arrest bleeding. However, the gelatin is a collagen based material from animal extract and contains heterogenetic protein which may cause anaphylaxis, resulting in feverish symptoms in patients. Further, the human body absorbs the gelatin sponge material slowly, and on average required more than four weeks to fully dissolve, which may cause patient tissue infection and wound healing retardation. Collagen sponges, which are also extracted from animal tissue, promote blood coagulation by activating endogenous coagulation cascade while also concentrating blood components by absorbing water in the blood.

Like the gelatin sponges, collagen sponges are also sourced from animal components and contain heterogenetic protein which is slowly absorbed in human body. The collagen sponge may produce complications as anaphylaxis, delayed wound healing and tissue infections. Due to these clinical risks, applications of collagen sponges may be limited.

### 2. Oxidized cellulose hemostatic gauze, oxidized regenerated cellulose hemostatic gauze:

Oxidized cellulose is a cellulose derivative. The hemostatic mechanism of oxidized cellulose is the concentration of blood components through the hygroscopic activity of oxidized cellulose, which stimulates blood coagulation as the carboxyl groups combine with haemoglobin Fe to produce acidic hematin in the blood. The forming clot seals capillary vessels and promotes hemostasis. Oxidized regenerated cellulose has the same mode of action as the oxidized cellulose.

Oxidized cellulose is synthetic. Normal human tissue degrades oxidized cellulose slowly by metabolizing enzymes. This process generally requires 3-6 weeks depending on the dosage and the tissue located in the body. Oxidized cellulose may cause local tissue infection and adversely affect local tissue healing process.

### 3. Fibrin glues

The Fibrin glues consist of fibrinogen, thrombin, aprotinin and calcium chloride. The hemostatic action relies mainly on the activation of fibrinogen by the thrombin to promote coagulation of cascade. Fibrin sealants are a mixture of fibrinogen and thrombin and have been widely used in recent years. The thrombin and fibrin in fibrin glue are sourced from either human or animal components and therefore create the risks of anaphylaxis and viral infections such as hepatitis, AIDS and BSE. Fibrin glues demonstrate weak adhesion when applied to wet, bleeding tissue and may be ineffective in the presence of active bleeding. Further, fibrin glues require special mixing, timing and storage condition.

### 4. Natural biological polysaccharide products

In recent years, natural, biological polysaccharide based products have focused much attention. The natural biological polysaccharide products are derived from plant material or chitosans which usually presented in powder format. These products have super biocompatibility, no toxicity, no tissue irritation, and no risk of anaphylaxis or viral infection from animal or human components contained in other hemostats.

### 1) Chitosan/chitin products:

Chitosan products are typically available in high swelling and non-absorbable sponges. Chitosan is made from the crushed shells of crustaceans. Chitosan has rapid hydrophilic capability and can activate the blood coagulation mechanism through its strong ionic charge. However, due to a lack of human enzymes to degrade chitosan, chitosan-derived products will be confined to topical applications. There is no evidence that chitosan products have been used in clinical as absorbable surgical hemostats to date.

### 2) Microporous Polysaccharide Hemospheres (MPH)

In 2002, MEDAFOR, INC. (USA) developed an absorbable hemostatic material called Arista™ (U.S. Patent No. US6060461), which consists of microporous polysaccharide Hemosphere (MPH). The MPHs are made through the reaction of purified starch and epichlorohydrin, wherein the epichlorohydrin reacts with starch molecules. This reaction results in the formation of ethyl propanetriol which creates a glucose molecule crosslink to the 3D network structure.

There are a few disadvantages of the MPH hemostatic powder. Firstly, the delivery of MPH mainly focuses on local, easy-to-access wound sites but presents some difficulties for effective applications for deep, tortuous wounds, in particular during the endoscopic procedures (such as minimally invasive surgery via endoscope and laparoscope). Secondly, during the production process, epichlorohydrin, a colourless, oily and toxic chemical is employed to produce a required reaction. This production is not environment friendly. Thirdly, the hemostatic efficacy of MPH is not satisfactory in particular for profuse bleeding due to its low hydrophilic capacity and slow water absorption characteristic. Fourthly, the adhesiveness of MPH to tissue is not strong enough following contacting with blood. The low viscosity, low adhesiveness of MPH following water absorption may reduce the hemostatic efficacy of MPH due to its weak sealing capability to wounded tissues and broken vessels. Fifthly, in the presence of active bleeding, the MPH powder can be easily washed away by blood flow if not compressed with a gauze on the top of powder. This gauze compression requirement adds an additional step in the hemostatic powder application technique and may risk re-bleeding when the gauze is removed. Therefore, MPH may have an unsatisfactory hemostatic efficacy for active bleeding.

### 3) Cellulose

China publication number CN1533751A discloses a hemostatic wound dressing, having a trade name of SURGICEL^{™}. SURGICEL^{™} includes a cellulose fabric and multi-porous polymer substrate on fabric surface which contacts the wound. The substrate contains biocompatible water soluble or water-soluble polymers. The fabric fibers are oxidized regenerated cellulose and the biocompatible, water-soluble polymers are polysaccharides. This hemostatic wound dressing consists primarily of oxidized cellulose, a slowly degradable material in the human body.

### Summary of the Invention

An object of the present invention is to provide a biocompatible modified starch hemostatic foam/sponge.

Another object of the present invention is to provide the new use of biocompatible modified starch hemostatic foam/sponge.

Another object of the present invention is to provide the production method to produce the said biocompatible modified starch hemostatic foam/sponge.

Another object of the present invention is to provide a kind of products of biocompatible modified starch hemostatic foam/sponge.

The present invention provides a biocompatible, modified starch composed hemostatic foam/sponge which can be topically applied to bleeding wound surface in humans and/or animals as a topical and/or surgical hemostat. Hemostasis occurs immediately and effectively when the said hemostat is in contact with blood on wound sites. The said hemostat can be degraded by human body. In addition, the said hemostatic foam/sponge can adhere the wound after contact with blood to seal the bleeding wound sites. It is easy to use. It can be used for many kinds of surgeries as surgical hemostat.

In addition, the said biocompatible modified starch hemostatic foam/sponge in the present invention can be used as an anti-adhesion material, a tissue healing promotion material, a surgical sealant and tissue adhesive glue to assist tissue repair. Further, the present invention is to provide a method to produce the said biocompatible modified starch hemostatic foam/sponge.

Accordingly, the said modified starch in the present invention includes one of the pregelatinized starch, acid modified starch, dextrin, oxidized starch, esterified starch, etherized starch, cross-linked starch, grafted starch, compound starch or their composition.

The starch modification process according to the present invention includes starch modified by physically, chemically, enzymatically, and naturally, or starch modified repeatedly with at least one of the above methods or a combination of two or more of the above methods.

The physical modifying process according to the present invention comprises irradiation, mechanical and steam treatment.

The chemical modifying process according to the present invention includes acidolysis, oxidation, esterfication, etherification, cross-linking, chemical agent grafting, or multiple modifying processes including at least two of the above processes, or one of the above modifying processes performed at least twice.

The described hemostatic composition comprises two or more modified starches to satisfy the physical and chemical properties of a hemostatic agent, where the weight ratio of the two modified starch groups can be 99:1 to 1:99.

Specifically, the weight ratio of the two modified starch groups can be: 95:5, 90:10, 85:15, 80:20, 75:25, 70:30, 65:35, 60:40, 55:45, or 50:50.

The said biocompatible modified starch hemostatic foam/sponge of the present invention has a water absorbency capacity not lower than one time its weight, whereas it can be 1-500 times generally and 5-100 times preferably.

The etherized starch of the present invention includes, but not limited to, at least one carboxymethyl starch, hydroxyethyl starch and cationic starch.

For example, the carboxymethyl starch is a polymer of linear structure as expressed in the following formula:

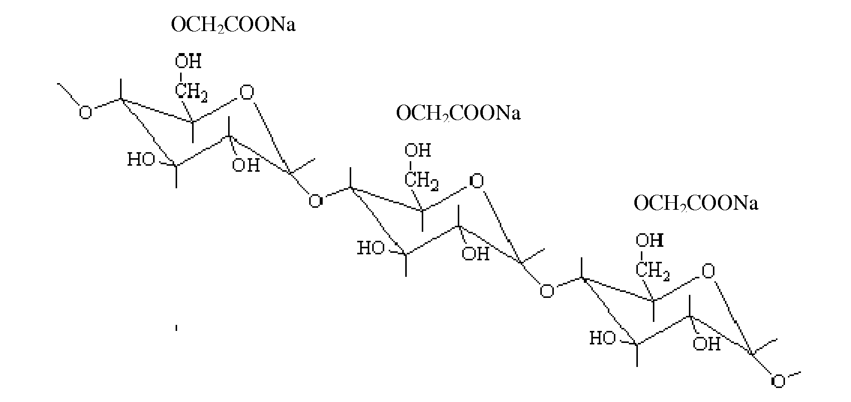

Modified starches such as carboxymethyl starch (CMS) and hydroxyethyl starch are known clinically as plasma substitutes. Carboxymethyl starch (CMS) and hydroxyethyl starch exhibit biocompatibility and safety with no toxic side effects when employed in the human circulatory system. The hemostatic formula, according to the present invention, can further include other plasma substitutes by means of well-known pharmacokinetics approaches and specified physical/chemical properties to produce safe and reliable hemostatic agents.

In order to enhance the efficacy of hemostasis, the cationic starch of the modified starch can be selected as hemostatic material. The surface positive charge of the cationic starch can attract and interact with electronegative blood erythrocytes to accelerate the blood coagulation process. Furthermore, when contacting with blood, the positively charged modified starch adheres tightly to tissue, seals the wound, and rapidly stops bleeding.

Multiple modification of modified starch comprises, but is not limited to, at least pre-gelatinized hydroxypropyl distarch phosphate. Specifically, the hydroxypropyl distarch phosphate is produced by cross-linking and etherifying the starch with propylene oxide and phosphoric acid, followed by pre-gelatinization modification through a spray drying process. The hydroxypropyl distarch phosphate of the present invention has both enhanced water absorbency capacity and high viscosity/adhesiveness, therefore has significant hemostatic capacity effect. It is stable in acidic or alkali environments and can be used as a biocompatible, hemostatic material, a surgical sealant, a tissue healing promotion agent, an anti-adhesion agent, and a tissue repair material.

The cross-linked starch of the present invention includes, but is not limited to, at least one of epichlorohydrin cross-linked starch and cross-linked carboxymethyl starch. The grafted starch of the present invention includes at least a propylene eater-carboxymethyl starch grafted copolymer and a crylic acid-carboxymethyl starch grafted copolymer. Grafted starch has both enhanced water absorption capability and high viscosity/adhesiveness. Therefore, it has a profound effect on hemostasis when applied to wound surfaces, especially combat wounds, traumatic wounds, and profuse bleeding from large arteries and large veins due to aneurysms ruptures.

Concerning active bleeding and high pressure arterial bleeding, surgeons and emergency responders must apply pressure to the wound to stop bleeding. In this circumstance, the hemostatic powder and the clot formed can be easily washed away by the high pressure blood flow resulting in a failure of hemosatsis. In addition, matrix of clot formed by blood and hemostatic particles easily sticks and adheres to surgical gloves, instruments and gauze. As a result, upon removal of gloves, instruments or gauze from the coagulated wound, re-bleeding may occur. The present invention provides a modified starch sponge/foam which can be applied and remain directly on the wound, thereby solving the above problem of re-bleeding. The modified starch hemostatic sponge/foam is absorbable, easy to use and may remain directly on the wound for a satisfactory effect.

In addition, the hemostatic sponge/foam of the present invention can be made into, but not limited to, columnar, sheet, massive, flocculent, or membranous form to meet surgical requirements.

The biocompatible modified starch hemostatic sponge/foam of the present invention can be produced by, but not limited, vacuum freeze drying.

The hemostatic foam of the present invention can be composite hemostatic foam made from one or more varieties of modified starches with other biocompatible hemostatic materials processed by vacuum freeze drying or other drying processes. Wherein, according to the present invention, the stated other biocompatible hemostatic materials comprise one or more of the groups of gelatin, collagen, carboxymethyl cellulose, oxidized cellulose, oxidize regenerated cellulose, and chitosan.

In order to solve the challenge of molding modified starch into sponge foam, the present invention combines other known bioabsorbable hemostatic materials having super biocompatibility and clinically acceptable qualities with the modified starch of the present invention to produce composite hemostatic sponges and foams. Whereas other known bioabsorbable hemostatic materials can be of one or more components, the modified starches can also be of one or more components, such as modified starch with gelatin, modified starch with collagen, modified starch with thrombin, modified starch with chitosan, modified starch with carboxymethyl cellulose, and modified starch with hyaluronic acid. These combinations can be molded into sponge/foam forms to satisfy clinical requirements.

Weight proportions between the said biocompatible modified starch and other biocompatible hemostatic materials can be 99:1 to 20:80.

Specifically, the weight ratio between the modified starch and other biocompatible hemostatic materials preferably is 95:5, 90:10. 80:20, 75:25, 70:30, 65:35, 60:40, 55:45, 50:50, 45:55, 40:60, 35:65, 30:70, 25:75, or 20:80.

Additional blood coagulant material may be added to the described modified starch hemostatic sponge or foam directly during the vacuum freeze drying production process to produce a composite hemostatic sponge/foam. The production process may involve, but is not limited to, pre-mixing the coagulant material with the modified starch directly before vacuum freeze drying process.

Accordingly, the coagulant of the present invention comprises one or more combinations of the following group of blood coagulation factors: thrombin, fibrin, calcium agent, polypeptide, peptide, amino acid, and protamine.

The modified starch sponge/foam of the present invention can be produced into hemostatic sponges and foam formed by a vacuum freeze drying process utilizing a forming agent or a plasticizing agent.

Whereas the forming agents of the present invention comprises, but not limited to, organic forming agents, inorganic forming agents, natural forming agents, and synthetic plasticizing agents which may include, but not limited to, one or more combinations of glycerol, kaolin, sorbitol, ethanol, ammonia, and polyethylene glycol. Specifically, the vacuum freeze drying process is a drying method that freezes wet material or solutions to a solid state under low temperatures (from -10°C to -50 °C) and then converts the solid material into a gas and then, in a vacuum (1.3-1.5 Pascal), back to a sold material without an intermediate liquid phase (sublimination). As the vacuum freeze drying is processed under low temperature and low pressure, the moisture sublimes directly to produce a substance with numerous special properties. The basic parameters of the vacuum freeze drying process specify both physical parameters and process parameters. The physical parameters include thermal conductivity, transfer coefficient, etc. The process parameters include freezing, heating, state of the material, etc. Continued research on this freezing process involves experiments to identify the optimal freezing curve.

According to the present invention, the topical application of modified starch can be used as a hemostatic agent to manage and control bleeding wound surfaces in humans, mammals, birds, or reptiles.

Furthermore, the biocompatible modified starch sponge/foam can be applied directly to bleeding wound site as a hemostat during the surgical operation and trauma treatments.

As the modified starch is applied as hemostatic material, other biological benefits of the modified starch are worthy of attention. It is essential to evaluate the effort of the biocompatible sponge/foam for its additional beneficial effects on wound inflammation, tissue adhesion and tissue healing while it is functional as a hemostat. It is proved by studies and experiments that this biocompatible modified starch, according to the present invention, has further applications as an absorbable, postoperative tissue adhesion barrier. The adhesion barrier of the modified starch, according to the present invention, prevents wounded tissue or organs from adhering to other tissue or organs in the vicinity by reducing local bleeding and exudation or by mechanically isolating the wound or wound surface from adjacent tissue and surrounding organs, such as the peritoneum.

The biocompatible hemostatic modified starch, according to the present invention, can promote tissue healing, including skin, subcutaneous soft tissue, musculature, bone tissue, neurological tissue, nerve tissue, and wounded tissue of the liver, kidney, spleen, etc. through proper dosage and application.

The mechanism for promoting tissue healing with said modified starch includes, but not limited to, the "glue" formation after modified starch contacts blood and establishes the "scaffold" established on the wound surface can facilitate the adherence, growth, connection and propagation of tissue cells such as osteoblasts or fibroblasts. In addition, local blood platelets are increasingly concentrated on the wound and, when activated, release tissue factors which promote healing.

The modified starch, according to the present invention, has further application as a biocompatible surgical sealant which is capable of forming a protective layer of colloid or film on the wound surface to seal and prevent drainage of blood, tissue fluids, lymph fluid, cerebrospinal fluid, bile, gastric fluid, and other intestinal fluids resulting from surgery and trauma treatment. This sealing effect will prevent lymph fistula leakage, bile flaccidity, pleural flaccidity, intestinal flaccidity, cerebrospinal flaccidity, and vascular flaccidity.

The modified starch, according to the present invention, has further application as biocompatible tissue adhesive glue which is capable of adhering, repairing, and bonding wounded nerve tissue, musculature, and tissues of the bone, skin, viscera, and subcutaneous tissue. It will also bond other curative materials to wounded tissue and organs.

The described biocompatible modified starch hemostatic sponge/foam can also be made into hemostatic film and hemostatic plaster to be applied directly to the bleeding site. The hemostatic film and plaster can be made into a film or an attaching layer to the inside or surface of a fiber fabric, such as a bandage or band-aid.

The described biocompatible modified starch hemostatic sponge can also be made into a hemostatic glue. The physical form can be colloidal, dissolved colloidal, thawed colloidal, semi-liquid or gelatinous, etc.

The stated hemostatic glue can be produced by adding other liquids, not limited to water, to the modified starch by diluting, swelling, or dissolving the liquids in certain proportions.

In addition to the above advantages, the modified starch of the present invention has an anti-inflammatory effect on bleeding wound. The modified starch hemostatic material, according to the present invention, has a hemostatic effect which controls bleeding, reduces blood and tissue fluid exudation, and maintains a moist wound surface. As a result, it suppresses the growth of bacteria and reduces the inflammatory response, diminishing local irritation and relieving and relieving pain. Furthermore, to strengthen the anti-inflammatory response, known antibiotic or other anti-pyrotic agents can be mixed with the modified starch during the manufacturing process to produce composite anti-inflammation and hemostatic powder, sponges and foams, hemostatic glues and gels, etc.

The modified starch sponge/foam has physical properties of pliability, flexibility, moldability, and curling. It can be adapted for wound surfaces with various shapes, sizes, and features, such as deep and irregular anatomical wounds, both inside and outside of the lacuna surface and organs, and can be applied via endoscope, laparoscope.

To enhance the safety of application in clinical use, the modified starch material, according to the present invention, can be packaged and sterilized with, but not limited to, gamma irradiation, oxirane, and ozone sterilization.

The alcohol disinfection, high temperature sterilizing and steam sterilizing is not recommended in the sterile process, because it is likely to change the physical characteristics of the modified starch, and results in weakening the efficacy of hemostasis.

The benefits of the present invention include:

The biocompatible modified starch hemostatic sponge/foam is easy to use and can be applied to the surgical procedure by compressing. It is effective to the active bleeding.

This present invention selects the biocompatible modified starch as the principle hemostatic material to be applied directly to the bleeding sites with the properties of strong biocompatibility, not toxicity, not tissue irritation, and not risk of anphylaxis or viral infection resulting from applying other animal/human components derived hemostat. Any fiber fabric material or animal component are not required to use in the present invention sponge/foam to minimize the risk of anaphylaxis, wound infection and wound healing retardation resulting from applying other animal/human components derived hemostats. The hydrophilic structure of the said sponge/foam can highly accelerates the water absorption.

When absorbing water, the biocompatible modified hemostatic sponge becomes super adhesive which can form a gelled matrix with blood to adhere and seal the wound and broken vessels, therefore the hemostatic efficacy is improved.

The modified starch hemostatic sponge/foam in the present invention is easy to use, low cost and biocompatible.

Another advantage of the modified starch hemostatic material of the present invention is the rapid particle dissolution in water, facilitating the easy removal of excess modified starch particles from the wound by simple saline irrigation. The residual modified starch not actively involved in hemostasis can be rinsed away by irrigation. In the treatment of battle wounds, self rescue or first aid, the hemostatic material remaining in small amounts will be degraded by the body and the irritation of wound debridement or gauze removal is avoided.

The modified starch hemostatic material has properties of stability, extended shelf life, resistance to high and low pressure, resistance to high temperature(up to 60 °C) and low temperature(down to - 40 °C), convenient storage, and physical stability. Therefore, it may also be employed as a hemostatic material for the military, emergency, and first-aid uses. Particularly, it can be adapted for extreme environmental conditions such as desert areas, polar regions, alpine areas, outer space, and underwater probes.

### Brief Description Of The Drawing

FIG. 1 illustrates a representative scanning electron microscope photo of section of hemostatic sponge A.
FIG. 2 illustrates a representative scanning electron microscope photo of section of hemostatic sponge B.

### Detailed of Description of the Preferred Embodiment

### Preferred Embodiment 1

Two gram 51# pre-gelatinized hydroxypropyl distarch phosphate is added into 30 ml water and stirred continuously to make starch particles swell sufficiently and disperse into a uniform suspension. Several drops of glycerol are added as a plasticizing agent (forming agent). The liquid is then put in a container and pre-cooled at -40 °C for 22 hours. It is then frozen and dried for 20 hours at -40 °C in a vacuum < 20 Pascal in a freezing drier. The final product is modified starch hemostatic sponge A.

Three gram hemostatic sponge A is added into 30ml water. The gelled matrix glue is formed, with its adherent capability of 76.42g.sec.

The stickiness of material in the present invention is measured as the work of adhesion using a texture analyzer (Physical Property Analyzer; Stable Micro System, Model TA-XT plus). Experiment conditions are: pre-experimental speed: 0.5 mm/sec; experimental speed: 10.0 mm/sec.

When the probe moves back, it will encounter the adhesive force produced by the sample. For the probe to separate completely from the sample, it must do work. The work done during this period is referred to as the work of adhesion and can be used to measure the adhesive strength (degree of firmness) between the adhesive agent and probe surface.

### Preferred Embodiment 2

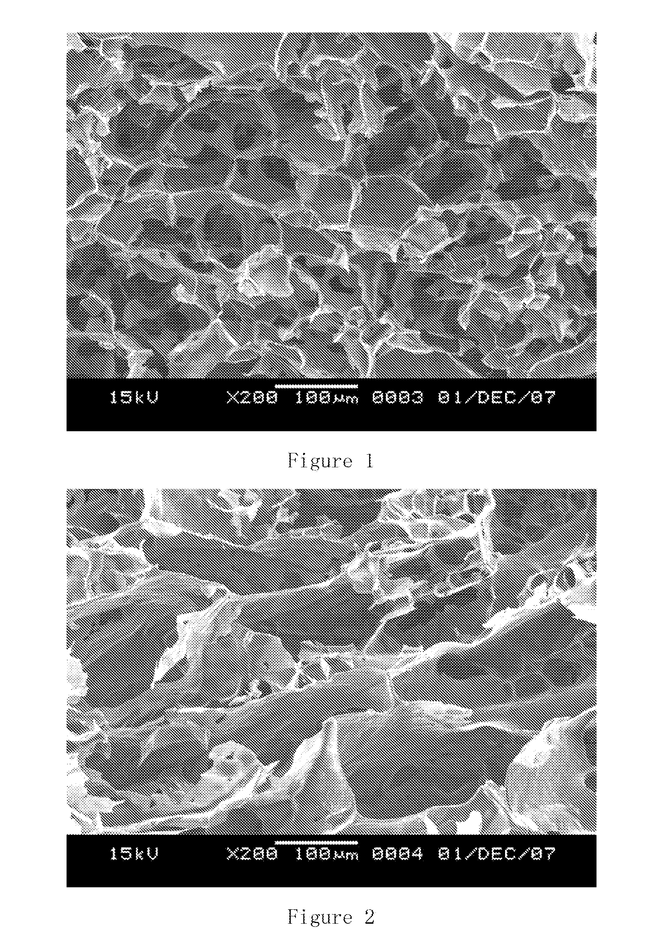

One gram 51# pre-gelatinized hydroxypropyl distarch phosphate is added into 30 ml water and stirred continuously to make starch particles swell sufficiently and disperse into a uniform suspension. The liquid is then put in a container and precooled at -40 °C for 22 hours. It is then frozen and dried for 20 hours at -50 °C in a vacuum < 20 Pascal in a freezing drier. The final product is a modified starch hemostatic sponge B. Referring to FIG. 1, a scanning electron microscope photo for the section of hemostatic sponge A is illustrated, and referring to FIG. 2, a scanning electron microscope a photo for the section of hemostatic sponge B is illustrated. Adding plasticizing agent during production can reduce sponge pore's diameters and enhance its density and specific surface area.

### Preferred Embodiment 3

Two gram 66# carboxymethyl starch is added into 30 ml water and stirred continuously to make starch particles swell sufficiently and disperse into a uniform suspension. The liquid is then put in a container and precooled at -40 °C for 22 hours. It is then frozen and dried for 20 hours at -50 °C in a vacuum < 20 Pascal in a freezing drier. The final product is modified starch hemostatic sponge C.

1.5 g 66# hemostatic sponge is added in 30ml water to create a uniform suspension gelled matrix glue. Its adherent capability is 162.68 g.sec.

### Preferred Embodiment4

Three gram 66# crosslinked carboxymethyl starch is added into 30 ml water and stirred continuously to make starch particles swell sufficiently and disperse into a uniform suspension. The liquid is then put in a container and precooled at -40 °C for 22 hours. It is then frozen and dried for 20 hours at -45 °C in a vacuum < 20 Pascal in a freezing drier. The final product is modified starch hemostatic sponge D.

### Preferred Embodiment 5

Three gram 88# hydroxyethyl starch is added into 30 ml water and stirred continuously to make starch particles swell sufficiently and disperse into a uniform suspension. The liquid is then put in a container and precooled at -40 °C for 22 hours. It is then frozen and dried for 20 hours at -50 °C in a vacuum <20 Pascal in a freezing drier. The final product is modified starch hemostatic sponge E.

### Preferred Embodiment 6

A certain amount of medical gelatin (10 g) is added into 100 ml water and heated in a beaker to 60 °C to form a colloidal solution. Two gram 66# carboxymethyl starch is added into 30 ml water and stirred continuously to make starch particles swell sufficiently and disperse into a uniform suspension. The two solutions are mixed together in a container with the mass ratio of medical gelatin to 66# carboxymethyl starch at 1:1. After precooling at -40 °C for 22 hours, it is frozen and dried for 20 hours under -45 °C in a vacuum < 20 Pascal in a freezing drier. The final product is modified starch composite hemostatic sponge F.

### Preferred Embodiment 7

A certain amount of medical gelatin (10 g) is added into 100 ml water and heated in a beaker to 60 °C to form a colloidal solution. Two gram 66# carboxymethyl starch is added into 30 ml water and stirred continuously to make starch particles swell sufficiently and disperse into a uniform suspension. The two solutions are mixed together in a container with the mass ratio of medical gelatin to 66# carboxymethyl starch at 2:1. After precooling at -40 °C for 22 hours, it is frozen and dried for 20 hours under -45 °C in a vacuum < 20 Pascal in a freezing drier. The final product is modified starch composite hemostatic sponge G.

### Preferred Embodiment 8

A certain amount of medical gelatin (10 g) is added into 100 ml water and heated in a beaker to 60 °C to form a colloidal solution. One gram 51# hydroxypropyl distarch phosphate is added into 30 ml water, and stirred continuously to make starch particles swell sufficiently and disperse into a uniform suspension. The two solutions are mixed together in a container with the mass ratio of medical gelatin to 51# hydroxypropyl distarch phosphate at 2:1. After precooling at 40 °C for 22 hours, it is frozen and dried for 20 hours under -45 °C in a vacuum < 20 Pascal in a freezing drier. The final product is modified starch composite hemostatic sponge H.

### Preferred Embodiment 9

A certain amount of medical gelatin (10 g) is added into 100 ml water and heated in a beaker to 60 °C to form a colloidal solution. One gram 51# hydroxypropyl distarch phosphate is added into 30 ml water and stirred continuously to make starch particles swell sufficiently and disperse into a uniform suspension. The two solutions are mixed together in a container with the mass ratio of medical gelatin to 51# hydroxypropyl distarch phosphate at 1:1. After precooling at -40 °C for 22 hours, it is frozen and dried for 20 hours under -45 °C in a vacuum < 20 Pascal in a freezing drier. The final product is modified starch composite hemostatic sponge I.

### Preferred Embodiment 10

One gram 66# carboxymethyl starch is added into 30 ml water and stirred continuously to make starch particles swell sufficiently and disperse into a uniform suspension. Then collagen is added gelatin and stirred symmetrically. The 66# carboxymethyl starch and the collagen mass ratio will be 5:1. The liquid is then put in a container and precooled at -40 °C for 22 hours. It is then frozen and dried for 20 hours at -50 °C in a vacuum < 20 Pascal in a freezing drier. The final product is modified starch composite hemostatic sponge K.

### Preferred Embodiment 11

One gram 51# hydroxypropyl distarch phosphate is added into 30 ml water and stirred continuously to make starch particles swell sufficiently and disperse into a uniform suspension. Then collagen gelatin is added and stirred symmetrically. The 51# hydroxypropyl distarch phosphate and the collagen mass ratio will be 5:1. The liquid is then put in a container and precooled at -40 °C for 22 hours. It is then frozen and dried for 20 hours at -50 °C in a vacuum < 20 Pascal in a freezing drier. The final product is modified starch composite hemostatic sponge L.

**Table 1**

| Comparison of Physics and Chemistry Characteristics of Modified Starch Hemostatic Sponges with Other Hemostatic Sponges | | | | |
|---|---|---|---|---|
| Sample | Density (Weight/ Volume) g/cm | Water Absorbency Capacity ( times of its weight) | Hydrophilic property | Water Absorption Velocity |
| Hemostatic Sponge A | 0.0679 | 19.7 | Hydrophilic, no Absorbing equilibrium contact angle* | Instantly |
| Hemostatic Sponge B | 0.0563 | 21.4 | Hydrophilic, no Absorbing equilibrium contact angle | Instantly |
| Hemostatic Sponge C | 0.0688 | 22.8 | Hydrophilic, no Absorbing equilibrium contact angle | Instantly |
| Hemostatic Sponge D | 0.0983 | 24.9 | Hydrophilic, no Absorbing equilibrium contact angle | Instantly |
| Hemostatic Sponge E | 0.11179 | 7.6 | Hydrophilic, no Absorbing equilibrium contact angle | Instantly |
| Absorbable Gelatin (Nan Jing Jin Ling Parma.) | 0.0099 | 40.6 | Slow. contact Extremely sponge angle is 106.degree | Extremely Slow |
| Collagen Sponge ( KROD Ltd., Beijing ) | 0.0235 | 33.2 | Hydrophilic, no Absorbing equilibrium contact angle | Slow |
| Oxidized Cellulose Hemostatic Gauze (J & J) | 0.0288 | 16.4 | Hydrophilic, no Absorbing equilibrium contact angle | Instantly |
| Chitosan Hemostatic Pad (HEMCON USA) | 0.1071 | 35.3 | Hydrophilic, no Absorbing equilibrium contact angle | Slow |

| | | | | |
|---|---|---|---|---|
| * An introduction to the measurement of contact angle | | | | |

Apparatus: OCA40Micro video contact angle measuring system (Dataphysics, Germany)
Methods: A sessile drop method was used to track and record water absorbing status of sponges using dynamic recording function and camera function. Details of the procedure were: a sponge sample was placed on an object table, and adjusted slowly to make the object stage appear at the inferior 1/3 portion of the visual field. A needle filled with deionized water connected with an injection unit was used. A drop of water with a certain volume was suspended at the tip of the needle using an automatic injection system. The system was focused so that the image of the sponge sample and water drop appeared clearly in the visual field. The object table was raised slowly so that the sponge sample touched the water drop. The camera function and dynamic recording function were turned on simultaneously to observe the process of water drop being absorbed and obtain the dynamic contact angle values.

**Table 2**

| The Water Absorbency Capacity of Composite Hemostatic Sponges | | | |
|---|---|---|---|
| | Volume of Water Absorption (ml) | Sample Weight (g) | Water Absorbency Capacity (times of its weight) |
| Composite Hemostatic Sponge F | 1.73 | 0.1 | 17.3 |
| Composite Hemostatic Sponge G | 1.95 | 0.1 | 19.5 |
| Composite Hemostatic Sponge H | 0.82 | 0.1 | 8.2 |
| Composite Hemostatic Sponge I | 1.1 | 0.1 | 11.0 |

Water absorbency of the sponges is determined by centrifugation. 0.025 g sponge was placed in 2 ml water, equilibrated for 10 minutes, and centrifuged for 10 minutes at 500 rpm. Sample was taken out, and weighed. The amount of remaining residual liquid was calculated. Each sample was measured 6 times. Average values were used. Volume density of sponges was measured. A sponge sample was cut into certain length and width and height, and weighed to calculate the density. Hygroscopicity and water absorbency of the sponges were observed through the OCA40Micro video contact angle measuring system of Dataphysics, Germany.

**Table 3**

| Water absorption velocity (speed) comparison of composite modified starch hemostatic sponges with other hemostatic sponges (Method of measurement: weight of all samples: 0.1 g, Measure the volume of water absorption in every 20 seconds following the start of water absorption) | | | | | | |
|---|---|---|---|---|---|---|
| | First 20 seconds (ml) | Second 20 seconds (ml) | Third 20 seconds (ml) | Forth 20 seconds (ml) | Fifth 20 seconds (ml) | Sixth 20 seconds (ml) |
| Composite Hemostatic Sponge F | 0.004 | 0.0035 | 0.0025 | 0.0025 | 0.0025 | 0.0025 |
| Composite Hemostatic Sponge G | 0.0035 | 0.0035 | 0.003 | 0.0025 | 0.0025 | 0.0025 |
| Composite Hemostatic Sponge H | 0.003 | 0.002 | 0.0007 | 0.0003 | 0.0003 | 0.0003 |
| Composite Hemostatic Sponge I | 0.0008 | 0.0008 | 0.0008 | 0.0005 | 0.0003 | 0.0003 |
| Absorbable Gelatin Sponge | 0.0008 | 0.0008 | 0.0005 | 0.0003 | 0.0003 | 0.0003 |
| Collagen Sponge | 0.0017 | 0.0008 | 0.0005 | 0.0003 | 0.0003 | 0.0003 |

As shown in Table 3, composite hemostatic sponge containing modified starch had significantly higher water absorbency capacity than gelatin sponge and collagen hemostatic sponge. Composite hemostatic sponge's maximal water absorbency capacity could reach 2 to 5 times higher than the pure gelatin sponge and collagen hemostatic sponge could. The composite modified starch hemostatic sponges can absorb water much faster and more efficiently than pure gelatin sponge and collagen sponge. And they can retain high water absorbency in the fifth and sixth 20 s than pure gelatin sponge and collagen sponge.

### Animal Experiment

Objective: To observe the hemostatic effect of modified starch hemostatic sponge in a liver bleeding models.

### Experimental Method:

A liver Tissue of an area of 2 cm.1 cm was cut off with a scalpel on the liver surface. Wound depth was 0.3 cm. Hemostatic sponges employed in the experiment to stop bleeding of the wound were:
1, 51# modified starch Hemostatic Sponge B;
2, 66# modified starch Hemostatic Sponge C;
3, composite hemostatic sponge I [51# modified starch: medical gelatin (mass ratio) = 1:1]
4, composite hemostatic sponge F [66# modified starch: gelatin (mass ratio) = 1:1]
5, composite hemostatic sponge K [66# modified starch: collagen (mass ratio) = 5:1]
6, composite hemostatic sponge L [51# modified starch: collagen (mass ratio) =5:1]
7, composite hemostatic sponge [66# modified starch: carboxymethyl cellulose (mass ratio) =1:1]

Pure gelatin sponge and collagen sponge were used as control groups. When the wound started to bleed, the hemostatic sponges were immediately placed on the wound site. A medical surgical glove or hemostatic gauze was used to be pressed on the wound to stop the blood stream. 1 to 2 min later, the glove or gauze was released gently. Observations of the study include the followings:
1) hemostatic effect in each groups
2) whether or not the surgical glove or gauze adhered to the sponge or the blood clot
3) Whether or not re-bleeding occurred when surgical glove or gauze was removed. It was unnecessary to remove modified starch sponge (experiment groups) after the bleeding was stopped. The wound was gently irrigated with saline after hemostasis achieved.

### Results:

All hemostatic sponges containing modified starch in the experimental groups had satisfactory hemostatic effect and were convenient to use. Sponges in the experimental groups can absorb moisture/blood immediately and form an adhesive sponge-blood coagulation colloid with the blood. Effective control of the bleeding from the liver wound was achieved in 1-2 minutes, in comparison to 3-5 min or more with the gelatin sponge and collagen sponge groups. Upon contact with the blood, hemostatic sponges in the experimental groups can adhere to the liver wound tissue tightly to promote blood coagulation and seal the bleeding vessels on the wound surface. The sponges in the experimental groups are elastic and easy to use. They do not adhere to the glove or gauze that are used to press the wound, and do not destroy the blood clot when the glove or gauze is removed, and therefore do not cause re-bleeding. The gelatin sponge and collagen hemostatic sponge in the control groups absorbed moisture/blood slowly, and needed to be pressed for multiple times. They adhered poorly to the tissue of wound surface, and had poor hemostatic effects.

## Claims

1. A kind of biocompatible modified starch sponge/foam.

2. The biocompatible modified starch sponge/foam, as recited in claim 1, wherein the modified starch sponge/foam is made to be used as one of the hemostatic material, anti-adhesion material, tissue healing promoting material, surgical sealant and tissue adhesive glue.

3. The biocompatible modified starch sponge/foam, as recited in claim 1, wherein, the modified starch includes one or the composite of pregelatinized starch, acid modified starch, oxidized starch, esterified starch, etherized starch, crosslinked starch, grafted starch and composite modified starch.

4. The biocompatible modified starch sponge/foam, as recited in claim 1, wherein its water absorbency capacity is no less than 1 time of its own weight.

5. The biocompatible modified starch sponge/foam, as recited in claim 4, wherein, the water absorbency capacity is preferable 5-100 times of its own weight.

6. The biocompatible modified starch sponge/foam, as recited in claim 3, wherein, the pregelatinized starch is produced by dry modification process or steam modification process, wherein dry modification process includes extrusion process and drum dry process; steam modification process includes spray drying process.

7. The biocompatible modified starch sponge/foam, as recited in claim 3, wherein, the etherized starch includes at least one of the carboxymethyl starch, hydroxyethyl starch and cationic starch.

8. The biocompatible modified starch sponge/foam, as recited in claim 3, wherein, the cross-linked starch includes at least one of the epichlorohydrin cross-linked starch and cross-linked carboxymethyl starch.

9. The biocompatible modified sponge/foam, as recited in claim 3, wherein, the multiple modification processed modified starch includes at least pregelatinized hydroxypropyl distarch phosphate.

10. The biocompatible modified starch sponge/foam, as recited in claim 3, wherein, the grafted starch includes at least one of the crylic acid-carboxymethyl starch grafting copolymer and propylene ester-carboxymethyl starch grafting copolymer.

11. The biocompatible modified starch sponge/foam, as recited in claim 1, wherein, the sponge/foam is in the form of columnar, sheer, massive, flocculent and membranous.

12. The biocompatible modified starch sponge/foam, as recited in claim 1, wherein, the method of production includes vacuum freeze drying process by freeze drying one or more modified starch.

13. The biocompatible modified starch sponge/foam, as recited in claim 1, wherein, the method of production includes vacuum freeze drying by freeze drying modified starch with other biocompatible hemostatic materials.

14. The biocompatible modified starch sponge/foam, as recited in claim 13, wherein, the other biocompatible hemostatic material comprises one or the combination of gelatin, collagen, carboxymethyl cellulous, chitosan and hyaluronic acid.

15. The biocompatible modified starch sponge/foam, as recited in claim 1, wherein, the method of production includes vacuum freeze drying process by freeze drying the modified starch with blood coagulant.

16. The biocompatible modified starch sponge/foam, as recited in claim 15, wherein, the blood coagulant comprises one or the combination of blood coagulation factors, fibrin, calcium agent, protamine, polypeptide and amino acid.

17. The biocompatible modified starch sponge/foam, as recited in claim 1, wherein, the method of production includes vacuum freeze drying process by freeze drying modified starch with plasticizer.

18. The biocompatible modified starch sponge/foam, as recited in claim 17, wherein, the plasticizer includes one or the combinations of glycerol, kaolin, sorbital, ethanol, ammonia and polyethylene glycol.

19. A method of treating bleeding wounds in mammals, birds and reptiles, comprising a step of applying biocompatible modified starch sponge/foam as recited in claim 1.

20. The method to apply the biocompatible modified starch sponge/foam, as recited in claim 19, further comprising a step of applying topically to bleeding wound tissue/organs to control bleeding during surgical operation, trauma treatment, self rescue and endoscopy.

21. The biocompatible modified starch sponge/foam, as recited in claim 1, wherein the biocompatible modified starch sponge/foam is in the form of hemostatic glue, hemostatic plaster and hemostatic film.
